# EUROPÄISCHE PATENTSCHRIFT

(11) **EP 0 397 951 B1**
(45) Veröffentlichungstag und Bekanntmachung des Hinweises auf die Patenterteilung: **20.07.1994**
(21) Anmeldenummer: 89710045.9
(22) Anmeldetag: 17.05.1989
(51) Int. Cl.: A61M 5/34

(54) **Spritze für medizinische Zwecke**
Syringe for medical purposes
Seringue à buts médicaux

(43) Veröffentlichungstag der Anmeldung: 22.11.1990
(73) Patentinhaber: Arzneimittel GmbH Apotheker Vetter & Co. Ravensburg, D-88212 Ravensburg (DE)
(72) Erfinder: Vetter, Helmut, D-7980-Ravensburg (DE); Geprägs, Peter, D-7987-Weingarten (DE)
(74) Vertreter: Fay, Hermann, Dipl.-Phys. Dr.

(56) Entgegenhaltungen:
- EP-A- 0 191 122
- WO-A-86/03126
- WO-A-88/00478
- US-A- 3 368 557
- US-A- 3 523 531
- US-A- 4 365 626

## Beschreibung

Die Erfindung betrifft eine Spritze für medizinische Zwecke, mit einem am Zylindermundstück des Spritzenzylinders aufgesetzten Nadelansatzstück, das unmittelbar oder über eine am Zylindermundstück eingerastete Überwurfkappe am Spritzenzylinder gehalten ist, wobei die Überwurfkappe das Nadelansatzstück außen dichtschließend ummantelt und aus einem im Vergleich zu dem gummielastischen Werkstoff des Nadelansatzstückes wesentlich steiferen Kunststoff besteht und dadurch das Nadelansatzstück versteift, und wobei die Überwurfkappe einen Anschlußhals zum Ansatz der Spritzennadel bildet, auf den bis zum Gebrauch der Spritze ein Tip-Cap als Verschlußteil aufgesetzt ist.

Derartige Spritzen sind aus der Praxis bekannt und z. B. in EP-A-0 191 122 die dem Oberbegriff des Anspruchs 1 zugrundeliegt, beschrieben. Sie kommen entweder als Fertigspritzen oder aber auch ungefüllt, in jedem Fall aber steril vorbehandelt zum Gebrauch. Sofern die Spritzen dabei nicht noch zusätzlich in eine Umhüllung eingeschlossen sind, ist der Spritze nicht anzusehen, ob sie sich noch in ihrem Originalzustand befindet oder ob beispielsweise das Tip-Cap zum Ansetzen der Kanüle bereits einmal entfernt gewesen ist, so daß sich die Spritze also nicht mehr in sterilem Zustand befindet. Damit eine Spritze nur in der Weise geöffnet werden kann, daß nachträglich erkennbar ist, ob die Spritze bereits einmal geöffnet worden ist, ist es aus der WO-A-8 603 126 bekannt, eine fest angeschlossene, aber abtrennbare Nadelschutzkappe vorzusehen. Diese Nadelschutzkappe umhüllt die Spritzennadel und erstreckt sich bis in den Bereich des Nadelansatzstückes. Zwischem dem Nadelansatzstück und dem der Nadelspitze zugeordneten Ende der Nadelschutzkappe ist eine durch eine Schwächungslinie gebildete Öffnungsvorrichtung angeordnet, bei der das der Nadelspitze zugeordnete Teil der Nadelschutzkappe von dem am Nadelansatzstück verbleibenden Teil entfernt werden kann.

Diese bekannte Lösung setzt aber voraus, daß die Kanüle bereits auf das Nadelansatzstück aufgesetzt ist, wobei ein Teil der Nadelschutzkappe die Kanüle am Nadelansatzstück hält.

Der Erfindung liegt die Aufgabe zugrunde, eine Spritze der eingangs genannten Art so auszubilden, daß sich ohne weiteres erkennen läßt, ob die Spritze sich noch in ihrem Originalzustand befindet oder aber bereits einmal geöffnet worden ist.

Diese Aufgabe wird nach der Erfindung dadurch gelöst, daß die Überwurfkappe eine fest angeschlossene, aber abtrennbar ausgebildete Sicherungskappe trägt, die den Anschlußhals sowie das Tip-Cap umschließt, wobei die Sicherungskappe aus einem im wesentlichen hohlzylindrischen Körper besteht, der an seinem axial einen Ende zumindest teilweise durch eine Stirnwand geschlossen ist und der an seinem anderen Ende einen stirnseitig vorstehenden, im Querschnitt hakenförmigen Kragen aufweist, der über einen dünnen Trennsteg mit der Sicherungskappe verbunden ist und in eine hinterschnittene Ringnut eines Sicherungsrings unlösbar einrastbar ist, wobei der Sicherungsring auf die Überwurfkappe fest aufgeschoben ist.

Der durch die Erfindung erreichte Vorteil besteht zunächst darin, daß die noch fest mit der Spritze verbundene Sicherungskappe bzw. deren Unversehrtheit einen zweifelsfreien Hinweis darauf gibt, daß die Spritze an ihrem nadelseitigen Ende noch nicht geöffnet worden ist, das Innere der Spritze also demzufolge nach wie vor steril ist.

Dies ist insbesondere auch dann von Vorteil, wenn die Überwurfkappe an ihrem dem Spritzenzylinder zugekehrten Ende mit über den Umfang gleichmäßig verteilt angeordneten Längsschlitzen versehen ist, so daß die zwischen den Längsschlitzen gebildeten Zungen zum Aufsetzen auf den Außenbund des Spritzenzylinders vorübergehend radial nach außen auffedern. Der über diese Zungen aufgeschobene Sicherungsring verhindert dann ein erneutes Lösen des Nadelansatzstückes bzw. der Überwurfkappe. Nach abschließender Konfektionierung und Aufsetzen des Tip-Cap kann dann die Sicherungskappe in dem Sicherungsring eingerastet werden. Dabei empfiehlt es sich weiter, daß der Sicherungsring für die Konfektionierung über einen dünnen Anschlußsteg unmittelbar an der Überwurfkappe des Nadelansatzstückes angeschlossen ist, wobei der Anschlußsteg einerseits mit der Außenschulter der sich zum Anschlußhals hin verjüngenden Überwurfkappe und andererseits mit dem innenseitigen Rand der Stirnfläche des Sicherungsrings verbunden ist. Hierdurch ist der Sicherungsring in einer für seine anschließende Montage geeigneten Lage an der Überwurfkappe angebracht, so daß nach deren Montage der Ring nur noch in axialer Richtung zum zylinderseitigen Ende unter Abtrennung des Anschlußsteges hin verschoben werden muß.

Weiter ist im Rahmen der Erfindung vorgesehen, daß der Sicherungsring die Überwurfkappe formschlüssig umgreift und in axialer Richtung durch ein zwischen dem Sicherungsring und der Überwurfkappe angreifendes Rastglied gehalten ist. Dieses Rastglied kann in bevorzugter Weise von einer an der Innenmantelfläche der Sicherungskappe vorgesehenen Ringnut und einem in die Ringnut einrastenden ringförmigen Vorsprung an der Sicherungskappe gebildet sein.

Ferner besteht die Möglichkeit, daß die Sicherungskappe mit einer stirnseitig eingebrachten axialen Bohrung versehen ist, deren lichter Durchmesser kleiner als der Außendurchmesser des Tip-Caps ist. Diese Bohrung ermöglicht es, im Wege einer Sichtkontrolle festzustellen, ob die Spritze ordnungsgemäß mit einem Tip-Cap versehen ist. Dennoch kann hierbei das Tip-Cap weder versehentlich noch beabsichtigt von dem Nadelansatzstück abgenommen werden. Hierbei empfiehlt es sich weiter, daß die Sicherungskappe mit ihrer Stirnfläche innenseitig dem Tip-Cap anliegt, so daß selbst ein axiales Bewegungsspiel des Tip-Caps ausgeschlossen ist. Schließlich kann vorgesehen sein, daß die Sicherungskappe sich zu ihrem stirnseitigen Ende hin kegelstumpfförmig verjüngt.

Im folgenden wird die Erfindung an einem in der Zeichnung dargestellten Ausführungsbeispiel näher erläutert; es zeigt:

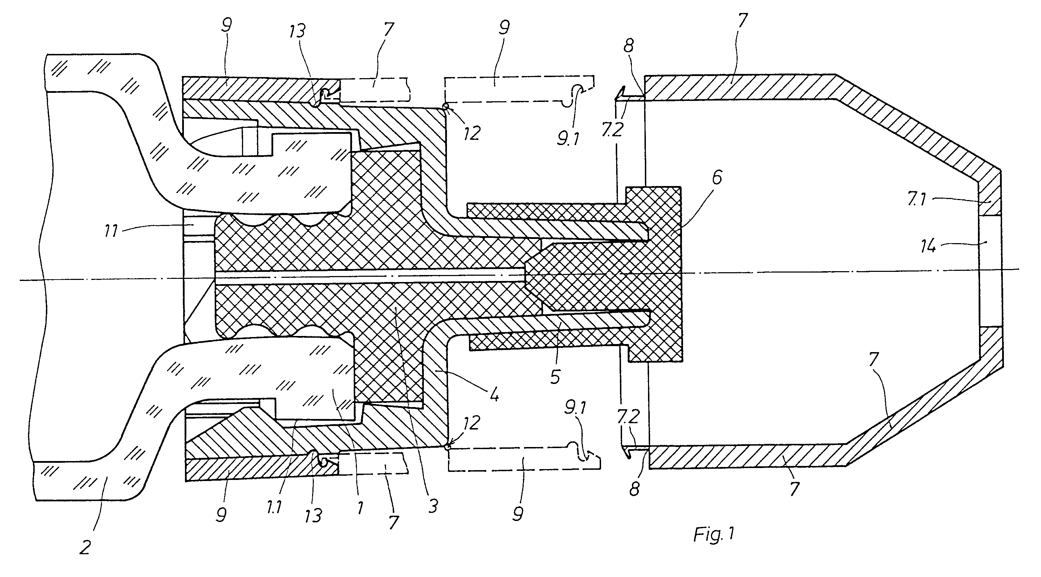

- Fig. 1: den Gegenstand nach der Erfindung mit nur teilweise dargestelltem Spritzenzylinder.

Die in der Zeichnung dargestellte Spritze ist zum Einsatz für medizinische Zwecke vorgesehen und trägt am Zylindermundstück 1 des Spritzenzylinders 2 ein aufgesetztes Nadelansatzstück 3, das über eine am Zylindermundstück 1 eingerastete Überwurfkappe 4 am Spritzenzylinder 2 gehalten ist. Die Überwurfkappe 4 bildet einen Anschlußhals 5 zum Ansatz einer in der Zeichnung nicht dargestellten Spritzennadel bzw. eines Tip-Cap 6, das vor Gebrauch der Spritze als Verschlußteil auf den Anschlußhals 5 aufgesetzt ist. Die Überwurfkappe 4 ummantelt außen dichtschließend das Nadelansatzstück 3 und besteht aus einem im Vergleich zu dem gummielastischen Werkstoff des Nadelansatzstückes 3 wesentlich steiferen Kunststoff, wodurch eine Versteifung des Nadelansatzstückes 3 erreicht wird.

Um vor Gebrauch der beispielsweise als vorgefüllte Fertigspritze oder auch unbefüllt, jedenfalls steril in den Verkehr kommenden Spritze sicher zu stellen, daß diese sich noch in ihrem Originalzustand befindet, trägt das Nadelansatzstück 3 eine abtrennbar ausgebildete Sicherungskappe 7, die den Anschlußhals 5 sowie das Tip-Cap 6 umschließt. Die Sicherungskappe 7 besteht im einzelnen aus einem im wesentlichen hohlzylindrischen Körper, der an seinem axial einen Ende zumindest teilweise durch eine Stirnwand 7.1 geschlossen ist. An dem anderen Ende weist die Sicherungskappe 7 einen stirnseitig vorstehenden, im Querschnitt hakenförmigen Kragen 7.2 auf, der über einen dünnen Trennsteg 8 mit der Sicherungskappe 7 verbunden ist. Dieser Kragen 7.2 ist in eine hinterschnittene Ringnut 9.1 des Sicherungsrings 9 unlösbar einrastbar, wobei der Sicherungsring 9 auf die Überwurfkappe 4 fest aufgeschoben ist. Diese Ausführungsform empfiehlt sich insbesondere dann, wenn das Nadelansatzstück 3 bzw. die Überwurfkappe 4 an ihrem dem Spritzenzylinder 2 zugewandten Ende mit in axialer Richtung verlaufenden Trennschnitten 11 versehen ist, durch die federnd aufspreizbare Zungen entstehen, die das Aufschieben der Überwurfkappe 4 auf den Außenbund 1.1 des Spritzenzylinders 2 erleichtern. Durch das anschließende Aufschieben des Sicherungsrings 9 wird ein erneutes Aufspreizen der Zungen und damit ein Abziehen des Nadelansatzstücks 3 von dem Spritzenzylinder 2 verhindert.

Um die Konfektionierung der Spritze zu vereinfachen, ist der Sicherungsring 9 über einen dünnen Anschlußsteg 12 unmittelbar an der Überwurfkappe 4 des Nadelansatzstückes 3 angeschlossen, wie dies in der Zeichnung gestrichelt angedeutet ist. Dabei ist der Anschlußsteg 12 einerseits mit der Außenschulter der sich zum Anschlußhals 5 hin verjüngenden Überwurfkappe 4 und andererseits mit dem innenseitigen Rand der Stirnfläche des Sicherungsrings 9 verbunden. Auf diese Weise kann der Sicherungsring 9 durch axialen Druck in Richtung zum Spritzenzylinder 2 montiert werden, der sich dabei durch Abtrennung des Anschlußsteges 12 löst, so daß es hierbei keiner Justierung des Sicherungsringes 9 bedarf.

Bei dieser Spritze wird die Überwurfkappe 4 formschlüssig von dem Sicherungsring 9 umgriffen, der in axialer Richtung durch ein zwischen dem Sicherungsring 9 und der Überwurfkappe 4 angreifendes Rastglied 13 gehalten ist. Dieses Rastglied 13 besteht im einzelnen aus einer an der Überwurfkappe 4 vorgesehenen Ringnut, in die ein an der Innenmantelfläche des Sicherungsrings 9 vorgesehener ringförmiger Vorsprung greift. Dieses Rastglied 13 ist in jedem Fall so ausgebildet, daß der Versuch, den Sicherungsring 9 von der Überwurfkappe 4 zu lösen, dazu führt, daß sich die Sicherungskappe 7 durch Einreißen des Trennsteges 8 vom Sicherungsring 9 löst.

Im übrigen ist die Sicherungskappe 7 stirnseitig mit einer axialen Bohrung 14 versehen, durch die eine nachträgliche Sichtkontrolle des Innenraums der Sicherungskappe 7 möglich ist, insbesondere das Vorhandensein des Tip-Caps 6 überprüft werden kann. Der lichte Durchmesser dieser Bohrung 14 ist dabei kleiner als der Außendurchmesser des Tip-Caps 6 gewählt, so daß dieses durch die Bohrung 14 hindurch nicht entfernt werden kann. Um auch jegliches axiales Spiel des Tip-Caps 6 auszuschließen, liegt die Sicherungskappe 7 mit ihrer Stirnfläche 7.1 innenseitig dem Tip-Cap 6 an.

Schließlich ist die Sicherungskappe 7 auch aus Gründen der Raumersparnis so ausgebildet, daß sie sich zu ihrem stirnseitigen Ende hin kegelstumpfförmig verjüngt.

## Patentansprüche

1. Spritze für medizinische Zwecke, mit einem am Zylindermundstück (1) des Spritzenzylinders (2) aufgesetzten Nadelansatzstück (3), das über eine am Zylindermundstück (1) eingerastete Überwurfkappe (4) am Spritzenzylinder (2) gehalten ist, wobei die Überwurfkappe (4) das Nadelansatzstück (3) außen dichtschließend ummantelt und aus einem im Vergleich zu dem gummielastischen Werkstoff des Nadelansatzstückes (3) wesentlich steiferen Kunststoff besteht und dadurch das Nadelansatzstück (3) versteift, und wobei die Überwurfkappe (4) einen Anschlußhals (5) zum Ansatz der Spritzennadel bildet, auf den bis zum Gebrauch der Spritze ein Tip-Cap (6) als Verschlußteil aufgesetzt ist, dadurch gekennzeichnet, daß die Überwurfkappe (4) eine fest angeschlossene, aber abtrennbar ausgebildete Sicherungskappe (7) trägt, die den Anschlußhals (5) sowie das Tip-Cap (6) umschließt, wobei die Sicherungskappe (7) aus einem im wesentlichen hohlzylindrischen Körper besteht, der an seinem axial einen Ende zumindest teilweise durch eine Stirnwand (7.1) geschlossen ist und der an seinem anderen Ende einen stirnseitig vorstehenden, im Querschnitt hakenförmigen Kragen (7.2) aufweist, der über einen dünnen Trennsteg (8) mit der Sicherungskappe (7) verbunden ist und in eine hinterschnittene Ringnut (9.1) eines Sicherungsrings (9) unlösbar einrastbar ist, wobei der Sicherungsring (9) auf die Überwurfkappe (4) fest aufgeschoben ist.

2. Spritze nach Anspruch 1, dadurch gekennzeichnet, daß der Sicherungsring (9) für die Konfektionierung über einen dünnen Anschlußsteg (12) unmittelbar an der Überwurfkappe (4) des Nadelansatzstückes (3) angeschlossen ist, wobei der Anschlußsteg (12) einerseits mit der Außenschulter der sich zum Anschlußhals (5) hin verjüngenden Überwurfkappe (4) und andererseits mit dem innenseitigen Rand der Stirnfläche des Sicherungsrings (9) verbunden ist.

3. Spritze nach den Ansprüchen 1 oder 2, dadurch gekennzeichnet, daß der Sicherungsring (9) die Überwurfkappe (4) formschlüssig umgreift und in axialer Richtung durch ein zwischen dem Sicherungsring (9) und der Überwurfkappe (4) angreifendes Rastglied (13) gehalten ist.

4. Spritze nach einem der Ansprüche 1 bis 3, dadurch gekennzeichnet, daß das Rastglied (13) von einer an der Innenmantelfläche der Sicherungskappe (7) vorgesehenen Ringnut und einem in die Ringnut einrastenden ringförmigen Vorsprung an dem Sicherungsring (9) gebildet ist.

5. Spritze nach einem der Ansprüche 1 bis 4, dadurch gekennzeichnet, daß die Sicherungskappe (7) mit einer stirnseitig angebrachten axialen Bohrung (14) versehen ist, deren lichter Durchmesser kleiner als der Außendurchmesser des Tip-Caps (6) ist.

6. Spritze nach einem der Ansprüche 1 bis 5, dadurch gekennzeichnet, daß die Sicherungskappe (7) mit ihrer Stirnfläche (7.1) innenseitig dem Tip-Cap (6) anliegt.

7. Spritze nach einem der Ansprüche 1 bis 6, dadurch gekennzeichnet, daß die Sicherungskappe (7) sich zu ihrem stirnseitigen Ende hin kegelstumpfförmig verjüngt.

## Claims

1. A syringe for medical purposes, comprising a needle attachment portion (3) which is fitted on the cylinder mouthpiece (1) of the syringe cylinder (2) and which is held on the syringe cylinder (2) by way of a retaining cap (4) which is latched on the cylinder mouthpiece (1), wherein the retaining cap (4) sealingly embraces the needle attachment portion (3) on the outside thereof and comprises a plastics material which is substantially stiffer in comparison with the elastic material of the needle attachment portion (3) and thereby stiffens the needle attachment portion (3), and wherein the retaining cap (4) forms a connecting neck (5) for attachment of the injection needle, on to which a tip cap (6) is fitted as a closure member until the syringe is used, characterised in that the retaining cap (4) carries a safeguard cap (7) which is fixedly connected but which is designed to be separable and which encloses the connecting neck (5) and also the tip cap (6), wherein the safeguard cap (7) comprises a substantially hollow-cylindrical body which at its axially one end is at least partially closed by an end wall (7.1) and which at its other end has a collar (7.2) which projects at the end wall of the body and which is of a hook-shaped cross-section and which is connected to the safeguard cap (7) by way of a thin separating limb (8) and is non-detachably engaged into an annular groove (9.1) of an undercut configuration in a safeguard ring (9), wherein the safeguard ring (9) is fixedly pushed on to the retaining cap (4).

2. A syringe according to claim 1 characterised in that for manufacture the safeguard ring (9) is connected by way of a thin connecting limb (12) directly to the retaining cap (4) of the needle attachment portion (3), wherein the connecting limb (12) is connected on the one hand to the outside shoulder of the retaining cap (4) which decreases towards the connecting neck (5) and on the other hand to the inside edge of the end face of the safeguard ring (9).

3. A syringe according to claim 1 or claim 2 characterised in that the safeguard ring (9) positively lockingly embraces the retaining cap (4) and is held in the axial direction by a retaining member (13) which engages between the safeguard ring (9) and the retaining cap (4).

4. A syringe according to one of claims 1 to 3 characterised in that the retaining member (13) is formed by an annular groove provided in the inside peripheral surface of the safeguard cap (7) and an annular projection on the safeguard ring (9), the projection engaging into the annular groove.

5. A syringe according to one of claims 1 to 4 characterised in that the safeguard cap (7) is provided with an axial bore (14) which is disposed at its end and the inside diameter of which is smaller than the outside diameter of the tip cap (6).

6. A syringe according to one of claims 1 to 5 characterised in that the safeguard cap (7) bears with its end face (7.1) at the inside thereof against the tip cap (6).

7. A syringe according to one of claims 1 to 6 characterised in that the safeguard cap (7) tapers in a frustoconical configuration towards its front end.

## Revendications

1. Seringue pour usage médical, comprenant un adaptateur d'aiguille (3) monté sur la tête (1) du cylindre de seringue (2) et maintenu directement ou par l'intermédiaire d'un capuchon protecteur (4) encliqueté sur la tête de cylindre (1), sur le cylindre de seringue (2), le capuchon protecteur (4) enveloppant extérieurement et de manière étanche l'adaptateur d'aiguille (3) et étant constitué d'une matière plastique beaucoup plus rigide en comparaison avec le matériau caoutchouteux de l'adaptateur d'aiguille (3) de façon à raidir ledit adaptateur d'aiguille (3), et le capuchon protecteur (4) formant un col de raccordement (5) pour la fixation de l'aiguille de la seringue, sur lequel est emboîté jusqu'à l'utilisation de la seringue, un tip-cap (6) qui sert d'élément de fermeture, **caractérisée en ce** que le capuchon protecteur (4) porte un capuchon de sécurité (7) fixe mais sectionnable qui enveloppe de col de raccordement (5) ainsi que le tip-cap (6), le capuchon de sécurité (7) étant constitué par un corps sensiblement cylindrique creux dont l'une des extrémités est obturée au moins en partie par une paroi frontale (7.1) et dont l'autre extrémité est munie d'un collet (7.2) de section crochue qui dépasse de la surface frontale, est relié au capuchon de sécurité (7) par l'intermédiaire d'une mince barrette de séparation (8) et peut s'enclencher de manière inamovible dans une rainure annulaire (9.1) contre-dépouillée d'une bague de sécurité (9), ladite bague de sécurité (9) étant emboîtée solidement sur le capuchon protecteur (4).

2. Seringue selon la revendication 1, caractérisée en ce que, pour le confectionnement, la bague de sécurité (9) est directement rattachée par l'intermédiaire d'une mince barrette de raccordement (12), au capuchon protecteur (4) de l'adaptateur d'aiguille (3), la barrette de raccordement (12) étant rattachée d'une part à l'épaulement extérieur du capuchon protecteur (4) qui se rétrécit en direction du col de raccordement (5) et, d'autre part, au bord intérieur de la surface frontale de la bague de sécurité (9).

3. Seringue selon l'une des revendications 1 ou 2, caractérisée en ce que la bague de sécurité (9) enveloppe le capuchon protecteur (4) à engagement positif et qu'elle est maintenue dans le sens axial par un élément d'encliquetage (13) agissant entre la bague de sécurité (9) et le capuchon protecteur (4).

4. Seringue selon l'une des revendications 1 à 3, caractérisée en ce que l'élément d'encliquetage (13) est constitué par une rainure annulaire prévue sur la surface latérale intérieure du capuchon de sécurité (7) et par une saillie annulaire formée sur la bague de sécurité (9) et s'enclenchant dans ladite rainure annulaire.

5. Seringue selon l'une des revendications 1 à 4, caractérisée en ce que le capuchon de sécurité (7) est muni d'un alésage axial (14) ménagé du côté frontal et dont le diamètre intérieur est plus petit que le diamètre extérieur du tip-cap (6).

6. Seringue selon l'une des revendications 1 à 5, caractérisée en ce que le capuchon de sécurité (7) est appliqué intérieurement, avec sa surface frontale (7.1), contre le tip-cap (6).

7. Seringue selon l'une des revendications 1 à 6, caractérisée en ce que le capuchon de sécurité (7) présente un rétrécissement tronconique en direction de son extrémité frontale.
